# EUROPEAN PATENT APPLICATION

(11) **EP 4 744 620 A2**
(43) Date of publication of application: **20.05.2026**
(21) Application number: 26160282.5
(22) Date of filing: 10.06.2021
(51) Int. Cl.: A61F 2/07

(54) **MULTI-BRANCH INTRALUMINAL DEVICES AND METHODS OF MAKING AND USING SAME**

(30) Priority: 10.06.2020 US 202063037115 P
(62) Divisional of application: 21737872.8
(71) Applicant: W. L. Gore & Associates, Inc., Newark, DE 19711 (US)
(72) Inventor: BIRDNO, Merrill J., Newark, Delaware, 19711 (US); KILGROW, Bret J., Newark, Delaware, 19711 (US); MAJOLAGBE, Kehinde Adeitunu, Newark, 19711 (US); WARD, Derek M., Newark, Delaware, 19711 (US); YOUNG, Patrick S., Newark, Delaware, 19711 (US)
(74) Representative: HGF

(57) **Abstract**

An implantable device comprising a main endoprosthesis configured for implantation in a vessel, the main endoprosthesis including at least one side portal, a first side-branch endoprosthesis configured to be deployed within the main endoprosthesis and direct flow through a first side-branch vessel via the at least one side portal, a second side-branch endoprosthesis configured to be deployed within the main endoprosthesis and direct flow through a second branch vessel via the at least one side portal, and wherein when not containing a side-branch endoprosthesis the at least one side portal comprises a side channel without fluid separation.

## Description

### CROSS REFERENCE TO RELATED APPLICATIONS

This application claims priority to Provisional Application No. 63/037,115, filed June 10, 2020 which is incorporated by reference in its entirety.

### FIELD OF THE INVENTION

The present invention relates to implantable endoluminal devices and particularly to implantable devices that provide access to multiple side-branch vessels.

### BACKGROUND

One example of an implantable endoluminal device employing at least one side-branch is described in U.S. Patent Number 8,556,961 to Quinn. In this example, a bifurcated intravascular stent graft comprises primary stent segments and a primary graft sleeve, forming a main fluid channel and having a side opening therethrough. An external graft channel formed on the primary graft sleeve has a first end communicating with the side opening and an open second end outside the primary graft sleeve, thereby providing a branch flow channel from the main channel out through the side opening and external graft channel. The primary stent segments and graft sleeve engage an endoluminal surface of a main vessel and form substantially fluid-tight seals. The stent graft further comprises a secondary stent graft, which may be positioned partially within the external graft channel, through the open second end thereof, and partially within a branch vessel. The secondary stent graft engages the inner surface of the external graft channel and the endoluminal surface of the branch vessel, thereby forming substantially fluid-tight seals.

Unrecognized and/or unaddressed problems with existing endoluminal devices that provide access to multiple side-branch vessels include: difficulties in managing and avoiding entanglement of multiple guidewires; difficulty in cannulation of branch vessels; challenges in manufacture of devices with multiple side portals; complexities with device positioning and deployments and clinician training; and excessive device profile.

The present invention addresses such deficiencies found in prior devices that provided access to multiple side-branch vessels.

### SUMMARY OF THE INVENTION

The present disclosure is directed to an implantable endoluminal device that provides improved access to multiple side-branch vessels. The device described employs a main endoprosthesis that includes at least one side portal. When not containing any side-branch endoprostheses, the side portal comprises a single channel without fluid separation. The device is configured to allow two or more side-branch endoprostheses to be deployed through the one portal.

According one example ("Example 1"), an implantable device includes a main endoprosthesis configured for implantation in a vessel, the main endoprosthesis including at least one side portal, a first side-branch endoprosthesis configured to be deployed within the main endoprosthesis and direct flow through a first side-branch vessel via the at least one side portal, and a second side-branch endoprosthesis configured to be deployed within the main endoprosthesis and direct flow through a second side-branch vessel via the at least one side portal. The implantable device further includes wherein when not containing a side-branch endoprosthesis, the at least one side portal comprises a side channel without fluid separation.

According to a second example ("Example 2"), the implantable device of Example 1 further includes a third side-branch endoprosthesis configured to be deployed within the main endoprosthesis and direct flow through a third branch vessel via the at least one side portal.

According a third example ("Example 3"), the implantable device of Example 2 further includes wherein the first side-branch endoprosthesis, second side-branch endoprosthesis and third side-branch endoprosthesis are deployed through a femoral access site.

According to a fourth example ("Example 4"), the implantable device of any of the preceding examples further includes wherein the main endoprosthesis includes a graft and stent component.

According to a fifth example ("Example 5"), the implantable device of Example 4 further includes wherein the graft comprises expanded polytetrafluoroethylene.

According to a sixth example ("Example 6"), the implantable device of Example 4 or Example 5 further includes wherein the stent component comprises nitinol.

According to a seventh example ("Example 7"), the implantable device of Example 2 further includes wherein at least one of the first side-branch endoprosthesis, second side-branch endoprosthesis, and third side-branch endoprosthesis is self-expanding.

According to an eighth example ("Example 8"), the implantable device of Example 2 further includes wherein at least one of the first side-branch endoprosthesis, second side-branch endoprosthesis, and third side-branch endoprosthesis is balloon-expandable.

According to a ninth example ("Example 9"), the implantable device of any one of the preceding examples further includes wherein the side channel includes a series of grooves.

According to a tenth example ("Example 10"), the implantable device of Example 9 further includes wherein the series of grooves correspond in number to the number of side-branch endoprostheses to be deployed in the side channel to aid in sealing between the side-branch endoprostheses.

According to an eleventh example ("Example 11"), the implantable device of Example 8 or Example 9 further includes wherein the series of grooves are provided in the side channel through use of a side portal adaptor.

According to a twelfth example ("Example 12"), an implantable device for implantation into at least one vessel includes a main endoprosthesis for implantation into a main vessel of the at least one vessel, the main endoprosthesis having at least one side portal communicating with a side channel, and a side portal adaptor configured for attachment to the main endoprosthesis within the side portal and extending through the side channel, the side portal adaptor comprising a series of grooves. The implantable device further includes at least one side-branch endoprosthesis extending through the side portal, the at least one side-branch endoprosthesis being received by at least one groove of the series of grooves.

According to a thirteenth example ("Example 13"), the implantable device of Example 12 further includes three side-branch endoprostheses extending through the side portal and each side-branch endoprosthesis being received by one of three grooves of the series of grooves.

According a fourteenth example ("Example 14"), the implantable device of Example 12 or Example 13 includes wherein the side portal adaptor is attached to the main endoprosthesis through one of at least bonding, adhesives, and a frictional fit.

According to a fifteenth example ("Example 15"), the implantable device of any one of Examples 12-14 further includes wherein the series of grooves are connected through a center conduit.

According to a sixteenth example ("Example 16"), a method of deploying an implantable device having a main endoprosthesis, a first side-branch endoprosthesis, and a second side-branch endoprosthesis, the main endoprosthesis comprising a side portal, wherein the first and second side-branch endoprostheses are configured to extend through the main endoprosthesis and the side portal, includes accessing an aorta and extending a plurality of guidewires into the aorta and at least two side vessels branching from the aorta. The method further includes cannulating a main aortic lumen of the aortic with at least one of the plurality of guidewires, advancing the main endoprosthesis over the at least one guidewire within the aorta, deploying the main endoprosthesis, advancing the first and second side-branch endoprostheses within the at least two side vessels, and deploying the first and second side-branch endoprostheses.

According to a seventeenth example ("Example 17"), the method of Example 16 further includes wherein three side vessels branch from the aorta and advancing the first and second side-branch endoprostheses includes advancing a third side-branch endoprosthesis.

According to an eighteenth example ("Example 18"), the method of Examples 16 or 17 further includes wherein advancing the main endoprosthesis over the at least one guidewire includes advancing the main endoprosthesis in an undeployed configuration.

According to a nineteenth example ("Example 19"), the method of any one of Examples 16-18 further include wherein the main endoprosthesis comprises a stent component that is self-expanding and deploying the main endoprosthesis includes self-expansion of the stent component.

According to a twentieth example ("Example 20"), the method of any one of Examples 16-19 further include wherein the at least first and second side-branch endoprostheses each comprise a stent component that is self-expanding and deploying the first and second side-branch endoprostheses includes self-expansion of the first and second side-branch endoprostheses.

### BRIEF DESCRIPTION OF THE DRAWINGS

The accompanying drawings are included to provide a further understanding of the invention and are incorporated in and constitute a part of this specification, illustrate embodiments of the invention, and together with the description serve to explain the principles of the invention.
Figure 1 is a top plan view of one embodiment of a main endoprosthesis, in accordance with some embodiments;
Figure 2 is a side view of the main endoprosthesis of Figure 1, in accordance with some embodiments;
Figure 3 is a cross-section view taken along line 3-3 of Figure 2, in accordance with some embodiments;
Figure 4 is a side view of one embodiment of a main endoprosthesis as described herein, with three side-branch endoprostheses mounted therein, in accordance with some embodiments;
Figure 5 is an end view of the main endoprosthesis of Figure 4, showing the three side-branch endoprostheses, in accordance with some embodiments;
Figure 6 is a side view of another embodiment of a main endoprosthesis as described herein, with three side-branch endoprostheses, in accordance with some embodiments;
Figure 7 is an end view of the main endoprosthesis of Figure 6, showing the three side-branch endoprostheses, in accordance with some embodiments;
Figure 8 is a side view of a side portal adapter configured to be mounted in a main endoprosthesis and receive side-branch endoprostheses, in accordance with some embodiments;
Figure 9 is an end view of the side portal adaptor shown in Figure 8, in accordance with some embodiments;
Figure 10 is a schematic representation of a patient's aorta, right and left brachial arteries, brachiocephalic artery, right subclavian artery, left common carotid artery, and left subclavian artery in preparation of providing access to implant the main endoprosthesis and side-branch endoprostheses, in accordance with some embodiments;
Figure 11 is the schematic representation of a patient's anatomy as shown in Figure 10 illustrating placement of guidewires used to implant the devices described herein, in accordance with some embodiments;
Figure 12 is a schematic representation of a patient's aortic arch, showing placement of embolic filters in the brachiocephalic artery and the left common carotid artery in preparation of implantation of the devices described herein, in accordance with some embodiments;
Figure 13 is a schematic representation of the patient's aortic arch as shown in Figure 12, illustrating positioning of an undeployed main endoprosthesis as described herein, in accordance with some embodiments;
Figure 14 is a schematic representation of the patient's aortic arch as shown in Figure 13, illustrating partial deployment of the main endoprosthesis, in accordance with some embodiments;
Figure 15 is a schematic representation of the patient's aortic arch as shown in Figure 14, illustrating cannulation of guidewires from the brachiocephalic artery and left common carotid artery into a portal in the main endoprosthesis, in accordance with some embodiments;
Figure 16 is a schematic representation of the patient's aortic arch as shown in Figure 15, illustrating a confirmation step to assure proper positioning of all guidewires prior to positioning of side-branch endoprostheses as described herein, in accordance with some embodiments;
Figure 17 is a schematic representation of the patient's aortic arch as shown in Figure 16, illustrating positioning of each of the three side-branch endoprostheses within the side portal in the main endoprosthesis, in accordance with some embodiments;
Figure 18 is a schematic representation of the patient's aortic arch as shown in Figure 17, illustrating deployment of the three side-branch endoprostheses in the main endoprosthesis and extending respectively into the brachiocephalic artery, left common carotid artery, and left subclavian artery, in accordance with some embodiments;
Figure 19 is a schematic representation of the patient's aortic arch as shown in Figure 18, illustrating removal of the delivery systems, in accordance with some embodiments;
Figure 20 is a schematic representation of the patient's aortic arch as shown in Figure 19, illustrating aspiration of each of the embolic filters to removal debris prior to embolic filter removal, in accordance with some embodiments;
Figure 21 is a schematic representation of the patient's aortic arch as shown in Figure 20, illustrating removal of the embolic filters, in accordance with some embodiments; and
Figure 22 is a schematic representation showing locations for final closure of each of the patient's access sites following the final procedural steps illustrated in Figure 21, in accordance with some embodiments.

### DETAILED DESCRIPTION

Persons skilled in the art will readily appreciate that various aspects of the present invention may be realized by any number of methods and apparatuses configured to perform the intended functions. Stated differently, other methods and apparatuses may be incorporated herein to perform the intended functions. It should also be noted that the accompanying drawing figures referred to herein are not all drawn to scale, but may be exaggerated to illustrate various aspects of the present invention, and in that regard, the drawing figures should not be construed as limiting.

Although the present invention may be described in connection with various principles and beliefs, the present invention should not be bound by theory.

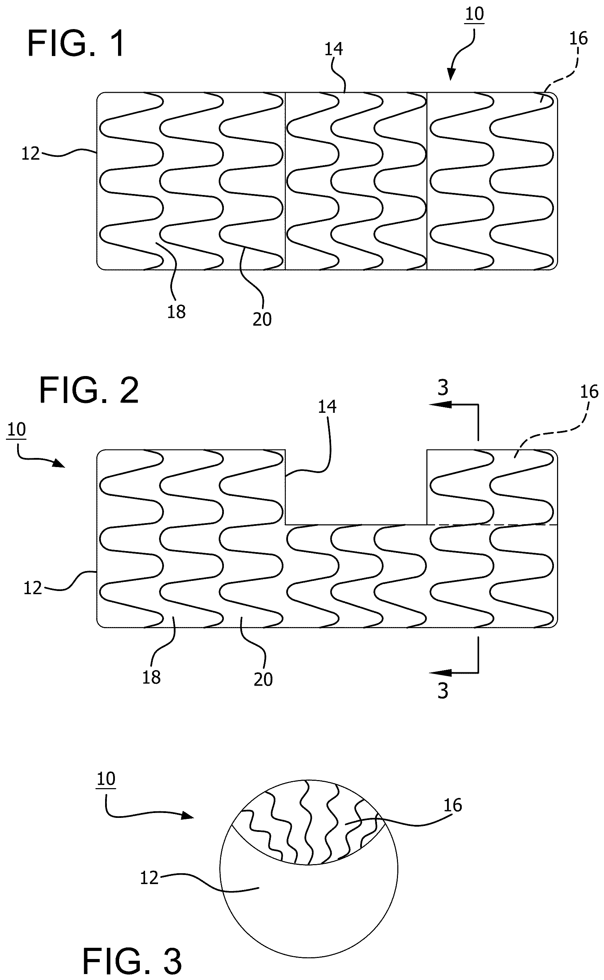

Figure 1, Figure 2, and Figure 3 illustrate an embodiment of an implantable device such as a main body endoprosthesis, also referred to as a main endoprosthesis 10, as described herein. Figure 1 is a top plan view of the embodiment of the main endoprosthesis 10. Figure 2 illustrates a side view of the main endoprosthesis 10 of Figure 1, and Figure 3 illustrates the cross sectional view taken along line 3-3 of the main endoprosthesis 10 of Figure 2.

The main endoprosthesis 10 includes a main flow lumen 12 and at least one side portal 14 communicating with a side channel 16. The main endoprosthesis 10 includes at least one graft component 18 and at least one stent component 20. In some embodiments, the main flow lumen 12 is an aortic lumen and may be described herein as a main aortic lumen 12. Each of the at least one graft components 18 and the at least one stent components 20 may be constructed from a variety of materials and through a variety of methods. Suitable materials, designs, and construction methods are disclosed, for example, in US Patents 5,476,589, 5,925,075, 7,691,141, 8,080,051, and 8,221,487, all incorporated herein by reference in their entireties. The at least one graft component 18 may be formed from a flexible biocompatible material, such as expanded polytetrafluoroethylene (ePTFE) or ultra-high molecular weight polyethylene (UHMWPE). The at least one graft component 18 may additionally be formed from a polyester such as Dacron. For self-expanding devices, the at least one stent component 20 may be formed from a shape-memory stent material, such as nitinol metal. For components where it is desirable that they be balloon-expandable or expandable by other expansion devices, a suitable stent material may comprise stainless steel or a similar malleable material.

The main endoprosthesis 10 is constructed so that it can be compacted to a smaller delivery configuration that allows it to be mounted on a catheter and remotely positioned in a patient's body. Once correctly positioned in a main vessel, such as an aorta, the main endoprosthesis 10 is deployed in the main vessel so as to seal a damaged area of the blood vessel and redirect blood flow around the damaged area through the main flow lumen 12. Deployment may be completed through balloon expansion of the main endoprosthesis 10 or through self-expansion of the main endoprosthesis 10.

If there are one or more branch blood vessels that will be covered by the main endoprosthesis 10 when deployed, a clinician can then align the side portal 14 with that vessel or vessels so as to allow perfusion therethrough via the side channel 16.

It is common, however, that damage in a main vessel may also extend to and into side-branch vessels. For example, in the case of an aneurysm in an aortic arch (not shown) of an aorta (not shown), weakening of the aorta may extend to one or more critical side-branch vessels, such as a patient's brachiocephalic artery, left common carotid artery, and/or left subclavian artery and sometimes beyond. In order to adequately repair an aortic aneurysm under such conditions, one or more additional endoprostheses may need to be deployed into the side-branch vessels to repair and maintain blood flow through those critical arteries.

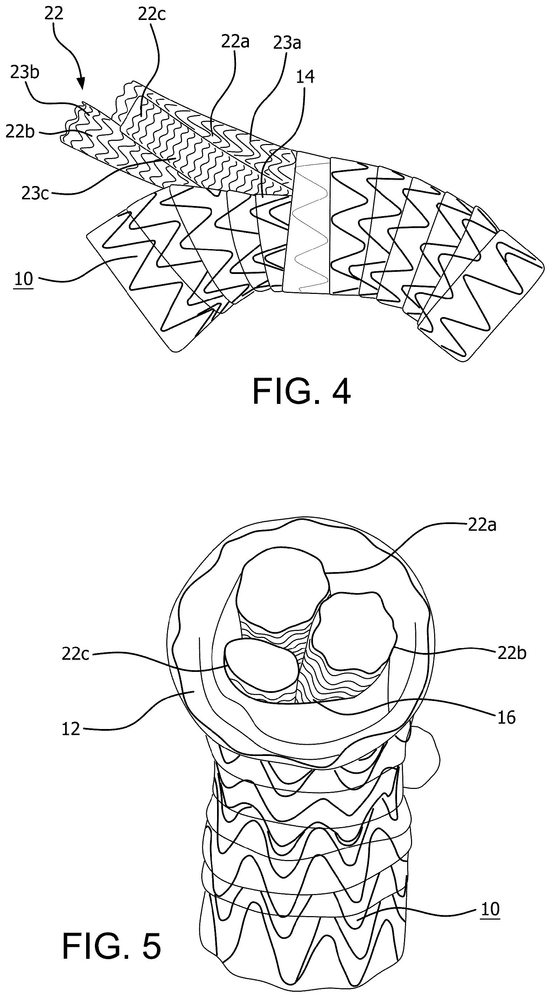

To accomplish such a repair, the main endoprosthesis 10 described herein is configured to allow one or more side-branch endoprostheses, as will be described with reference to Figure 4, to be deployed within the side channel 16, and extend into a targeted side-branch vessel via the side portal 14. In this manner, the side-branch endoprosthesis can effectively exclude damage in the main vessel and the side-branch vessels while assuring continued flow and perfusion of blood into the side-branch vessel. Side portals have been proposed and successfully implemented in the past (e.g., US Patent 8,556,961 to Quinn, incorporated by reference). However, it can often be a challenge to deliver multiple side-branch endoprostheses through multiple side portals, with clinicians having to manipulate and avoid entangling a plurality of guidewires, each corresponding to different side-branch vessels passing through multiple side portals in a main body endoprosthesis. Also, a challenge with multiple side portals is that there is often a huge variation in the exact location of branch vessels, thus making it difficult to align the main endoprosthesis 10 in a location that will allow perfusion into each branch vessel.

These concerns are various examples of challenges that may be solved by an embodiment disclosed herein. To address these concerns, the main endoprosthesis 10 employs the side portal 14 and deploys multiple side-branch endoprostheses into the side channel 16, as well be described further with reference to Figures 4 and 5.

Figure 4 is a side view of an additional embodiment of an implantable device including the main endoprosthesis 10 as described herein, with at least one side-branch endoprosthesis 22 mounted therein. Figure 5 is an end view of the main endoprosthesis 10 of Figure 4. The main endoprosthesis 10 has a first side-branch endoprosthesis 22a, a second side-branch endoprosthesis 22b, and a third side-branch endoprosthesis 22c, each deployed in the side channel 16 and extending through the side portal 14. The side portal 14 may be larger in diameter than the diameter of the at least one side-branch endoprosthesis 22 described herein.

Additionally, as illustrated in Figures 4 and 5, the first, second, and third side-branch endoprostheses 22a, 22b, 22c in this embodiment are each of different construction and each is configured to deploy into different side-branch vessels, such that a flow is directed through the first, second, and third side-branch endoprostheses 22a, 22b, 22c. Each of the first, second and third side-branch endoprostheses 22a, 22b, 22c has an outer surface 23a, 23b, 23c, respectively. A side surface of each of the outer surfaces 23a, 23b, 23c may additionally be referred to as a side wall. In some embodiments, the first, second and third side-branch endoprostheses 22a, 22b, 22c vary in respective diameters. For example, the first side-branch endoprosthesis 22a may have a larger diameter than the third side-branch endoprosthesis 22c. In other examples, the first, second and/or third side-branch endoprostheses 22a, 22b, 22c may have substantially equal diameters. In various embodiments, the first, second, and third side-branch endoprostheses 22a, 22b, 22c may comprise a stent component and/or a graft component similar to the stent component 20 (Figure 2) or the graft component 18 (Figure 2) of the main endoprosthesis 10.

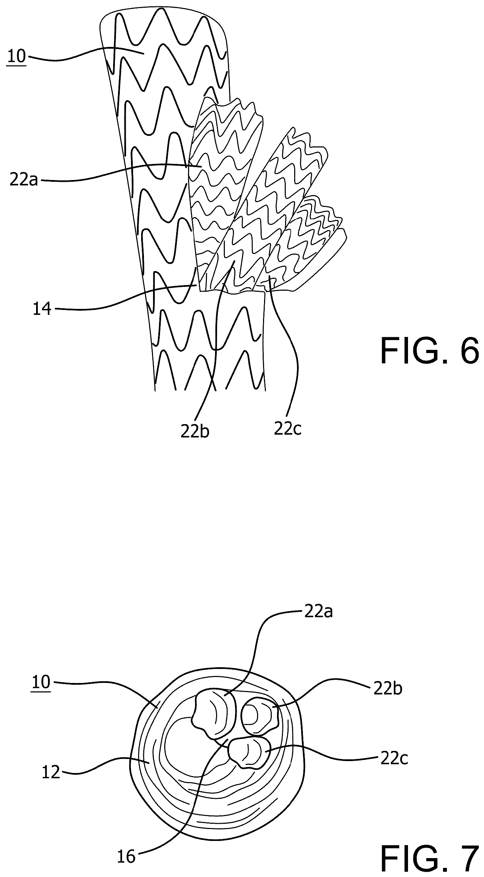

Another embodiment of the main endoprosthesis 10 is shown in Figures 6 and 7. Figure 6 is a side view of the main endoprosthesis 10 including the first, second, and third side-branch endoprostheses 22a, 22b, 22c deployed and extending out of the side portal 14. Figure 7 is an end view of the main endoprosthesis 10 with the first, second and third side-branch endoprostheses 22a, 22b, 22c deployed inside the side channel 16 of the main endoprosthesis 10.

It should be appreciated that the nature of this design allows for exceptional versatility in use. If no damage is located around the side-branch vessels, the main endoprosthesis 10 can be deployed with no side-branch endoprostheses which allows for perfusion through the side-branch vessels via the side channel 16 and the side portal 14. Alternatively, one, two, three, or more side-branch endoprostheses can be deployed as needed to provide repair and reperfusion of the side-branch vessels as may be required. For example, if four side-branch vessels require repair and reperfusion, four side-branch endoprostheses may be implanted and extend through the side portal 14.

By utilizing only the side channel 16, the various guidewires can be more easily manipulated by clinicians and the various guidewires are less likely to become entangled among each other while the main endoprosthesis 10 and the first, second, and third side-branch endoprostheses 22a, 22b, 22c are being positioned for deployment. Additionally, by providing the side portal 14 such that it is enlarged, it is expected that clinicians will more easily be able to cannulate guidewires through the side portal 14. This is believed to save time, effort, and frustration in performing procedures. Ease in cannulation is also believed to facilitate simpler through-and-through device positioning and delivery.

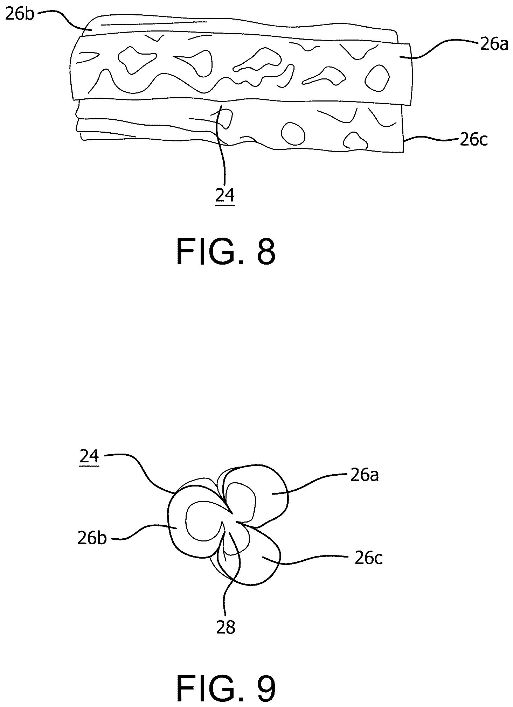

One concern with placing multiple side-branch devices, or side-branch endoprostheses, through a single channel is that endoleaks may occur, at least initially, in any gaps that may form between the side-branch endoprostheses. One embodiment which may address the concern is illustrated in the embodiment of Figures 8 and 9. Figure 8 illustrates a side view of a side portal adapter 24 configured to be mounted in the main endoprosthesis 10 (Figure 4) and receive the at least one side-branch endoprosthesis 22 (Figure 4). Figure 9 illustrates an end view of the side portal adapter 24. In this embodiment, the side portal adapter 24 includes a series of grooves 26 configured for receiving the first, second, and third side-branch endoprostheses 22a, 22b, 22c to be deployed in a given procedure, and a center conduit 28 is positioned at the center of the side portal adapter 24.

In this embodiment, and with reference to at least Figures 6 and 9, wherein the main endoprosthesis 10 is used in combination with the first, second and third side-branch endoprostheses 22a, 22b, 22c, the series of grooves 26 of the side portal adapter 24 comprises a first groove 26a for receiving the first side-branch endoprosthesis 22a, a second groove 26b for receiving the second side-branch endoprosthesis 22b and a third groove 26c for receiving the third side-branch endoprosthesis 22c. The side portal adapter 24 may be provided with a predetermined number of grooves 26 corresponding to the number of side-branch endoprostheses 22 to be deployed in a given procedure and is not meant to be limited to the numbers described herein. In order to at least avoid the guidewire entanglement concerns, such as those previously described, each of the series of grooves 26 is open to each other via the center conduit 28, allowing fluid communication (and thus guidewire movement) among the series of grooves 26, when there are no side-branch endoprostheses, or devices, deployed in the side portal adapter 24.

The side portal adapter 24 may be built into the construction of the main endoprosthesis 10 (Figure 6) or it may be formed separately, as shown in Figures 8 and 9, such that it can be placed into the side portal 14 (Figure 6) and the side channel 16 (Figure 7) after the main endoprosthesis 10 is constructed. The side portal adaptor 24 may be constructed from the methods of materials previously described with reference to the graft component 18 (Figure 2) and the stent component 20 (Figure 2) of the main endoprosthesis 10. The side portal adaptor 24 may be attached to the main endoprosthesis 10 with adhesives, bonding, frictional fit or any other suitable methods of attachment.

It should be appreciated that with or without the side portal adapter 24, other methods of sealing around the at least one side-branch endoprosthesis 22 may also be used to reduce or eliminate blood leakage, including, but not limited to, the following: configuring the side-branch endoprostheses 22a, 22b, 22c (Figure 4) to complement each other so as to form a tight fit along their respective outer surfaces 23a, 23b, 23c (Figure 4) when deployed; providing sealing cuffs, inflatable or expandable seals, adhesive, hydrogels, or other sealing mechanisms around the at least one side-branch endoprosthesis 22; providing sealing cuffs, inflatable or expandable seals, adhesive, hydrogels, or other sealing mechanisms in or around the side portal 14, the side channel 16, and/or the side portal adapter 24; introducing a sealing material into any gaps following deployment of the at least one side-branch endoprosthesis 22; coating one or more surfaces of the devices with a coagulation agent or similar substance that could induce blood to form a seal; or other similar concepts that may later be developed.

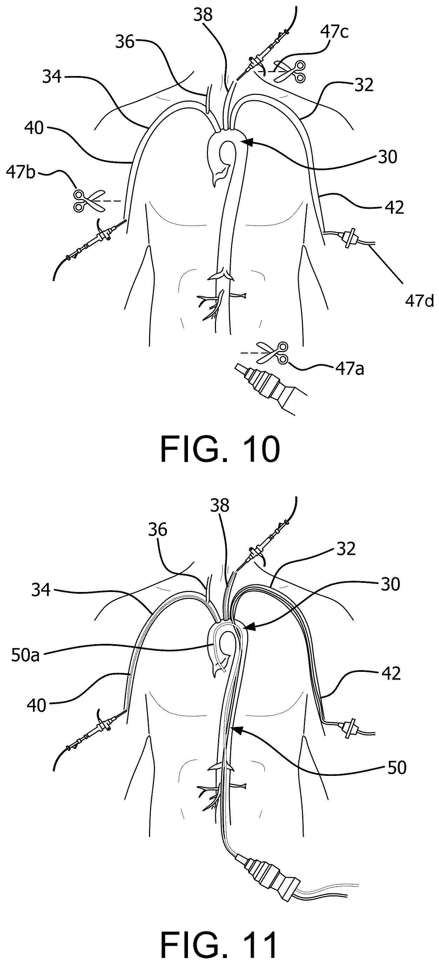

One embodiment of a deployment sequence of the implantable device, the main endoprosthesis 10 and the first, second and third side-branch endoprostheses 22a, 22b, 22c described herein, is illustrated in Figs. 10-22 and will be described therein and as follows:

Figure 10 is a schematic representation of a patient's aorta 30, right brachial artery 40, left brachial artery 42, brachiocephalic artery 36, right subclavian artery 34, left common carotid artery 38, and left subclavian artery 32, in preparation of providing access to implant the main endoprosthesis (not shown) and at least one side-branch endoprosthesis (not shown) described herein. Fig. 10 additionally illustrates where a clinician may create incisions at a plurality of access sites 47 for delivering the main endoprosthesis 10. The plurality of access sites 47 includes a first access site 47a, a second access site 47b, a third access site 47c, and a fourth access site 47d. The plurality of access sites 47 may also be described herein as a femoral access site 47a, a right brachial access site 47b, a carotid access site 47c, and a left brachial access site 47d.

Figure 11 is the schematic representation of the patient's anatomy as shown in Figure 10, further illustrating placement of a plurality of guidewires 50 used to implant the main endoprosthesis 10 and the first, second, and third side-branch endoprostheses 22a, 22b, 22c (Figure 6) described herein. As illustrated in Fig. 11, the plurality of guidewires 50 may be delivered into the patient's aorta 30, right brachial artery 40, left brachial artery 42, right subclavian artery 34, left common carotid artery 38, and left subclavian artery 32. As such, the plurality of guidewires 50 comprises an aortic guidewire 50a configured for deployment in the patient's aorta 30. In some embodiments, the plurality of guidewires 50 comprise more than one aortic guidewire 50a. Each of the plurality of guidewires 50 is characterized as having a rail, the rail being the outer surface of the respective guidewire. The plurality of guidewires 50 are illustrated prior to cannulation, and therefore may be described during this state as "pre-cannulated guidewires."

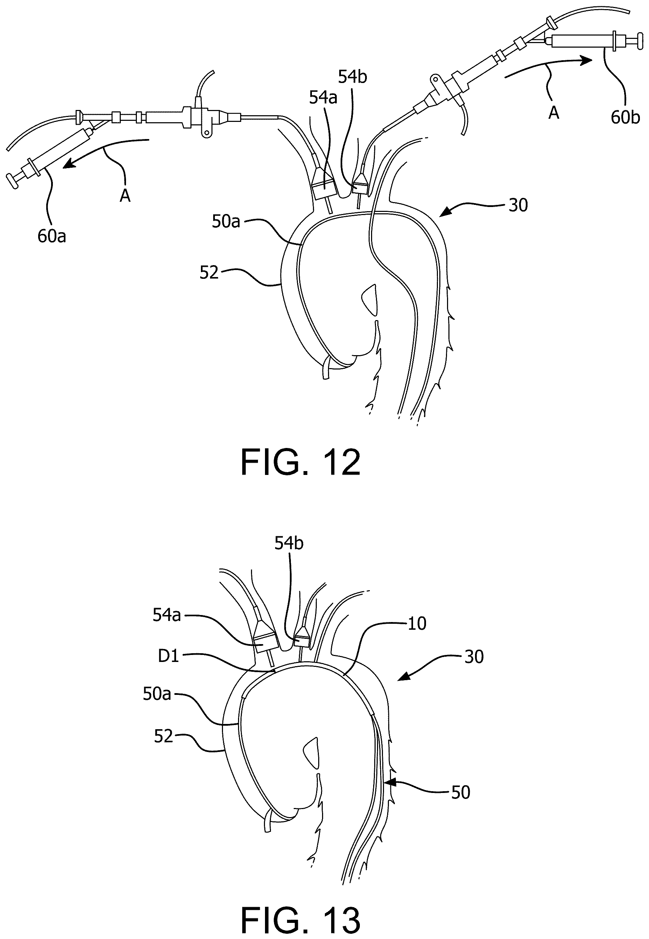

Figure 12 is a schematic representation of the patient's aorta 30, specifically an aortic arch 52, showing placement of a first embolic filter 54a in the brachiocephalic artery 36 (Figure 10) and a second embolic filter 54b in the left common carotid artery 38 (Figure 10) in preparation of implantation of the main endoprosthesis (not shown) and the side-branch endoprostheses (not shown) described herein. In various embodiments, there may be more than two embolic filters 54a, 54b delivered. Additionally, Figure 12 illustrates the use of a first syringe 60a and a second syringe 60b for intermittent flushing of fluids throughout the delivery procedure. Various fluids may be removed in the direction of arrows A using the first and second syringes 60a, 60b. In various embodiments, there may be more than two embolic filters 54a, 54b delivered.

Figure 13 is a schematic representation of the patient's aorta 30, specifically the aortic arch 52, as shown in Figure 12, further illustrating the positioning of the main endoprosthesis 10 when undeployed, as described herein. When in the undeployed configuration, the main endoprosthesis 10 is illustrated positioned around the aortic guidewire 50a and has a first diameter D1.

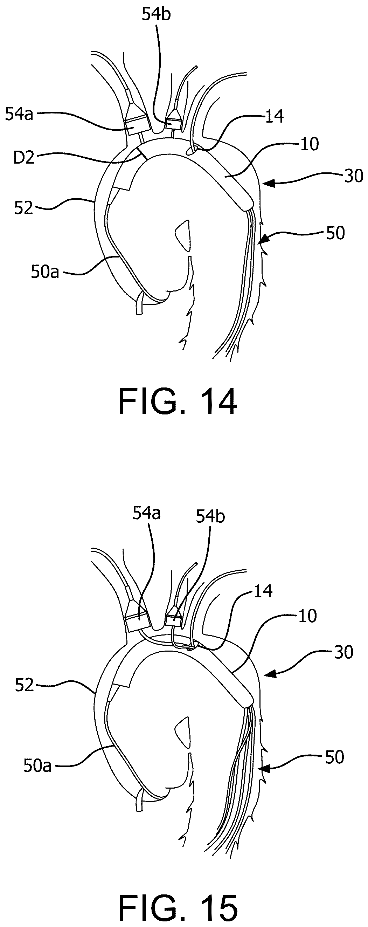

Figure 14 is a schematic representation of the patient's aortic arch 52 as shown in Figure 13, further illustrating partial deployment of the main endoprosthesis 10. As illustrated in this embodiment, in a partially deployed configuration the main endoprosthesis 10 has expanded in comparison to the undeployed configuration, wherein it comprises a second diameter D2 that is larger than the first diameter D1 (Figure 12) of the main endoprosthesis 10 in the undeployed configuration.

Figure 15 is a schematic representation of the patient's aortic arch 52 as shown in Figure 14 comprising the main endoprosthesis 10 in a partially deployed configuration, further illustrating cannulation of various of the plurality of guidewires 50 from the brachiocephalic artery 36 (Figure 10) and left common carotid artery 38 (Figure 10) into the side portal 14 of the main endoprosthesis 10.

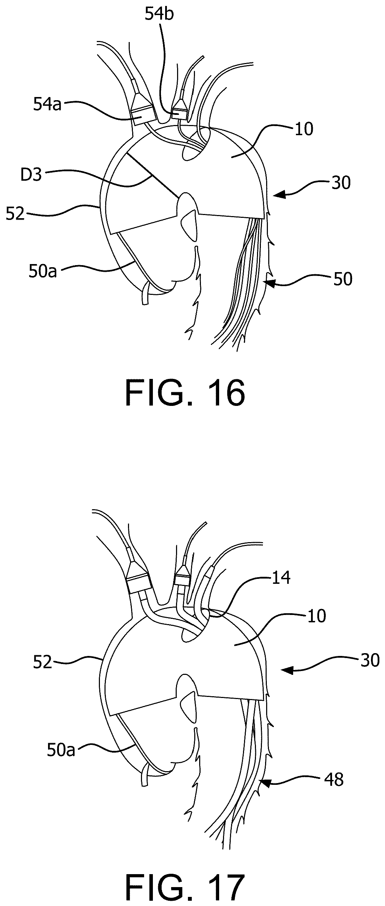

Figure 16 is a schematic representation of the patient's aortic arch 52 as shown in Figure 15, illustrating a confirmation step to assure proper positioning of each of the plurality of guidewires 50 prior to positioning of the side-branch endoprostheses (not shown) as described herein. As illustrated in Figure 16, the main endoprosthesis 10 has been expanded to a fully deployed configuration. This is defined by the main endoprosthesis 10 having a third diameter D3. In some embodiments, the third diameter D3 is larger than each the first and second diameters D1, D2, respectively, as illustrated in Figures 13 and 14. The deployment of the main endoprosthesis 10 may include balloon expansion of the main endoprosthesis 10 such that it expands to a desired diameter, or allowing the self-expanding stent component 20 (Figure 2) of the main endoprosthesis 10 to expand to a predetermined desired diameter. The desired diameter may vary in various embodiments depending on several factors such as, but not limited to, creating a sufficient seal between an outer surface of the main endoprosthesis 10 and the aortic arch 52, the anatomy of the patient, and the extent of damage to the vessels of the aorta 30.

Figure 17 is a schematic representation of the patient's aortic arch 52 of the aorta 30 as shown in Figure 16, illustrating positioning of each of the first, second and third side-branch endoprostheses within various side-branch endoprosthesis delivery systems 48 within the side portal 14 of the main endoprosthesis 10. The first, second, and third side-branch endoprostheses (not shown) have been advanced into the patient's aorta 30 from the femoral access site 47a (Figure 10) into the side portal 14 of the main endoprosthesis 10. In this embodiment, the first, second, and third side-branch endoprostheses are in an undeployed configuration, positioned over the plurality of guidewires 50 (Figure 16), and within the delivery systems 48. In this illustrative embodiment, the side-branch endoprosthesis delivery systems 48 are shown advanced from the femoral access site 47a of the patient, extending into the main endoprosthesis 10 and comprising a cap portion extending within each of the side-branch vessels.

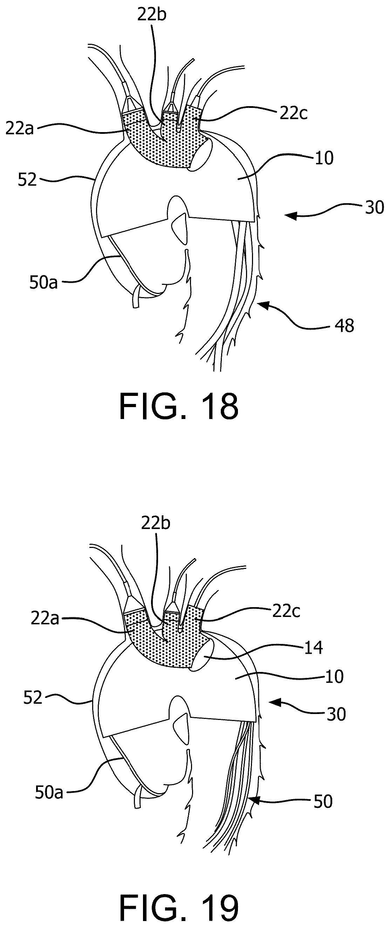

Figure 18 is a schematic representation of the patient's aortic arch 52 as shown in Figure 17, illustrating the deployed first, second, and third side-branch endoprostheses 22a, 22b, 22c in the main endoprosthesis 10. The first, second, and third side-branch endoprostheses 22a, 22b, 22c extend into the brachiocephalic artery 36 (Figure 11), the left common carotid artery 38 (Figure 11), and the left subclavian artery 32 (Figure 11), respectively. In the illustrative embodiment of Figure 18, the first, second, and third side-branch endoprostheses 22a, 22b, 22c are shown in a deployed state, wherein similar to the main endoprosthesis 10 deployed state, the diameter of each of the first, second, and third side-branch endoprostheses 22a, 22b, 22c is greater than their respective diameter in the undeployed configuration of Figure 17. In various embodiments, the first, second, and third side-branch endoprostheses 22a, 22b, 22c transition from the undeployed state to the deployed state through balloon expanding the first, second, and third side-branch endoprostheses 22a, 22b, 22c or allowing each of the first, second, and third side-branch endoprostheses 22a, 22b, 22c to self-expand until it reaches the desired size. The desired size may vary based on several factors such as, but not limited to, the size of the respective artery that the side-branch endoprosthesis is positioned within and the extent of the damage to the artery. In some embodiments, the first side-branch endoprosthesis 22a within the brachiocephalic artery 36 is deployed first, the second side-branch endoprosthesis 22b within the left common carotid artery 38 is deployed second, and the third side-branch endoprosthesis 22c within the left subclavian artery 32 is deployed third.

Figure 19 is a schematic representation of the patient's aortic arch 52 as shown in Figure 18, illustrating the removal of the side-branch endoprosthesis delivery systems 48 (Figure 18) such that towards the femoral access site 47a (Figure 10) only the guidewires 50 remain. In various embodiments, the sealings of the main endoprosthesis 10 and at least one side-branch endoprosthesis 22 are tested after the removal of the side-branch endoprosthesis delivery systems 48.

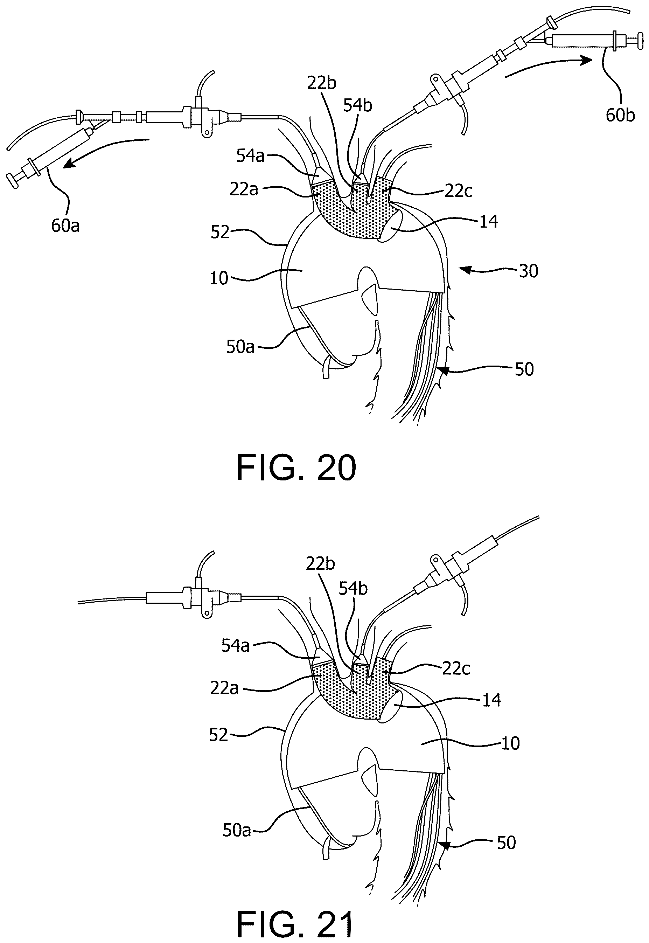

Figure 20 is a schematic representation of the patient's aortic arch 52 as shown in Figure 19 illustrating aspiration of each of the embolic filters 54a, 54b for removal of debris prior to removing the embolic filters 54a, 54b. The first and second syringes 60a, 60b may be used for the aspiration of each of the first and second embolic filters 54a, 54b. In various embodiments, any applicable method of aspirating the first and second embolic filters 54a, 54b may be used by the clinician.

Figure 21 is a schematic representation of the patient's aortic arch 52 as shown in Figure 20, illustrating removal of the embolic filters 54a, 54b.

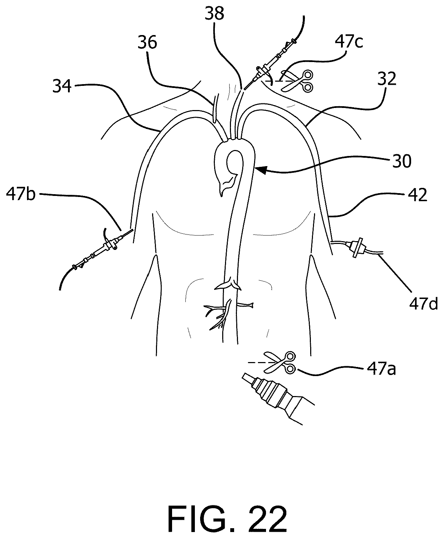

Figure 22 is a schematic representation showing locations for final closure of the patient's plurality of access sites 47 following the final procedural steps illustrated in Figure 21. In this embodiment, the main endoprosthesis (not shown) has been deployed, the plurality of guidewires 50 removed, and the appropriate closure of vessels completed.

To reiterate, a suitable process for deployment of the devices described herein in the aortic arch 52, and with reference to Figs. 6-22, may comprise the following:
1. Pre-cannulate the left subclavian artery with the plurality of guidewires 50 ("pre-cannulated guidewires") to achieve through-and-through access. Place embolic filters 54a, 54b as appropriate;
2. Cannulate the main aortic lumen 12 with the aortic guidewire 50a;
3. Advance the main endoprosthesis 10 upon the rail of the aortic guidewire 50a and pre-cannulated guidewires 50;
4. Partially deploy the main endoprosthesis 10 into the aorta 30. The side portal 14 for the side-branch vessels will remain patent and blood flow will be established through the main aortic lumen 12;
5. Cannulate the brachiocephalic artery 36 and the left common carotid artery 38;
6. Fully deploy the main endoprosthesis 10;
7. Advance the first, second, and third side-branch endoprostheses 22a, 22b, 22c from the femoral access site 47a. Deploy the first, second and third side-branch endoprostheses 22a, 22b, 22c one by one. Remove the side-branch endoprosthesis delivery systems 48. Ensure that the plurality of guidewires 50 are still in the same position as during the prior step;
8. Balloon the three side-branch endoprostheses 22a, 22b, 22c as appropriate to ensure good seal;
9. Test for sealing;
10. Aspirate any emboli from the embolic filters 54a, 54b; and
11. Remove the embolic filters 54a, 54b and plurality of guidewires 50 and close all access sites 47.

An additional suitable process for deployment of the devices described herein in the aortic arch 52, and with reference to Figs. 6-22, may comprise the following:
1. Pre-cannulate the branch vessels with the plurality of guidewires 50 ("pre-cannulated guidewires") to achieve through-and-through access. Place embolic filters 54a, 54b as appropriate;
2. Cannulate the main aortic lumen 12 with the aortic guidewire 50a;
3. Advance the main endoprosthesis 10 upon the rail of the aortic guidewire 50a and pre-cannulated guidewires 50;
4. Deploy the main endoprosthesis 10 into the aorta 30. The side portal 14 for the side-branch vessels will remain patent and blood flow will be established through the main aortic lumen 12;
5. Advance the first, second, and third side-branch endoprostheses 22a, 22b, 22c from the femoral access site 47a. Deploy the first, second and third side-branch endoprostheses 22a, 22b, 22c one by one. Remove the side-branch endoprosthesis delivery systems 48. Ensure that the plurality of guidewires 50 are still in the same position as during the prior step;
6. Balloon the three side-branch endoprostheses 22a, 22b, 22c as appropriate to ensure good seal;
7. Test for sealing;
8. Aspirate any emboli from the embolic filters 54a, 54b; and
9. Remove the embolic filters 54a, 54b and plurality of guidewires 50 and close all access sites 47.

While a benefit of the devices described herein is that multiple side-branch endoprostheses 22 can be delivered through the side portal 14, it should be appreciated that there may be applications where it is desirable to construct a device with multiple portals to address variations in anatomies and/or to treat multiple conditions with a single device. Accordingly, the main endoprosthesis 10 may be provided with multiple side portals, with some accommodating one or more additional endoprostheses, without departing from the concepts described herein.

Without intending to limit the scope of the present invention, among the benefits of the described intraluminal device include, without limitation, the following: negates the impact of wire wrap or other guidewire entanglement; easier cannulation of the branch vessels; fewer manipulations of the device in-vivo, which will reduce procedure time and effort and may avoid medical complications such as a stroke; ease of manufacture due to only a single side portal and channel needed; reduced complexity of the device and procedure allowing for simplified physician training; and less material required for fabricating the main endoprosthesis, allowing for a lower profile during delivery, among others.

It will be apparent to those skilled in the art that various modifications and variations can be made in the present invention without departing from the spirit or scope of the invention. Thus, it is intended that the present invention cover the modifications and variations of this invention provided they come within the scope of the appended claims and their equivalents.

The disclosure is also described with reference to the following numbered clauses:
Clause 1: An implantable device comprising:
   a main endoprosthesis configured for implantation in a vessel, the main endoprosthesis including at least one side portal;
   a first side-branch endoprosthesis configured to be deployed within the main endoprosthesis and direct flow through a first side-branch vessel via the at least one side portal;
   a second side-branch endoprosthesis configured to be deployed within the main endoprosthesis and direct flow through a second side-branch vessel via the at least one side portal; and
   wherein when not containing a side-branch endoprosthesis the at least one side portal comprises a side channel without fluid separation.
Clause 2: The implantable device of clause 1, further comprising a third side-branch endoprosthesis configured to be deployed within the main endoprosthesis and direct flow through a third branch vessel via the at least one side portal.
Clause 3: The implantable device of clause 2, wherein the first side-branch endoprosthesis, second side-branch endoprosthesis and third side-branch endoprosthesis are deployed through a femoral access site.
Clause 4: The implantable device of any one of the preceding clauses, wherein the main endoprosthesis comprises a graft and a stent component.
Clause 5: The implantable device of clause 4, wherein the graft comprises expanded polytetrafluoroethylene.
Clause 6: The implantable device of clauses 4 or 5, wherein the stent component comprises nitinol.
Clause 7: The implantable device of clause 2, wherein at least one of the first side-branch endoprosthesis, second side-branch endoprosthesis, and third side-branch endoprosthesis is self-expanding.
Clause 8: The implantable device of clause 2, wherein at least one of the first side-branch endoprosthesis, second side-branch endoprosthesis, and third side-branch endoprosthesis is balloon-expandable.
Clause 9: The implantable device of any one of the preceding clauses, wherein the side channel includes a series of grooves.
Clause 10: The implantable device of clause 9, wherein the series of grooves correspond in number to the number of side-branch endoprostheses to be deployed in the side channel to aid in sealing between the side-branch endoprostheses.
Clause 11: The implantable device of clauses 8 or 9, wherein the series of grooves are provided in the side channel through use of a side portal adaptor.
Clause 12: An implantable device for implantation into at least one vessel, the device comprising:
   a main endoprosthesis for implantation into a main vessel of the at least one vessel, the main endoprosthesis having at least one side portal communicating with a side channel;
   a side portal adaptor configured for attachment to the main endoprosthesis within the side portal and extending through the side channel, the side portal adaptor comprising a series of grooves; and
   at least one side-branch endoprosthesis extending through the side portal, the at least one side-branch endoprosthesis being received by at least one groove of the series of grooves.
Clause 13: The implantable device of clause 12, wherein the implantable device comprises three side-branch endoprostheses extending through the side portal, and each side-branch endoprosthesis is received by one of three grooves of the series of grooves.
Clause 14: The implantable device of clauses 12 or 13, wherein the side portal adaptor is attached to the main endoprosthesis through one of at least bonding, adhesives, and a frictional fit.
Clause 15: The implantable device of any one of clauses 12-14, wherein the series of grooves are connected through a center conduit.
Clause 16: A method of deploying an implantable device, the implantable device comprising a main endoprosthesis, a first side-branch endoprosthesis, and a second side-branch endoprosthesis, the main endoprosthesis comprising a side portal, wherein the first and second side-branch endoprostheses are configured to extend through the main endoprosthesis and the side portal, the method comprising:
   accessing an aorta;
   extending a plurality of guidewires into the aorta and at least two side vessels branching from the aorta;
   cannulating a main aortic lumen of the aortic with at least one of the plurality of guidewires;
   advancing the main endoprosthesis over the at least one guidewire within the aorta;
   deploying the main endoprosthesis;
   advancing the first and second side-branch endoprostheses within the at least two side vessels; and deploying the first and second side-branch endoprostheses
Clause 17: The method of clause 16, wherein three side vessels branch from the aorta and advancing the first and second side-branch endoprostheses includes advancing a third side-branch endoprosthesis.
Clause 18: The method of clauses 16 or 17, wherein advancing the main endoprosthesis over the at least one guidewire includes advancing the main endoprosthesis in an undeployed configuration.
Clause 19: The method of any one of clauses 16-18, wherein the main endoprosthesis comprises a stent component that is self-expanding and deploying the main endoprosthesis includes self-expansion of the stent component.
Clause 20: The method of any one of clauses 16-19, wherein the at least first and second side-branch endoprostheses each comprise a stent component that is self-expanding and deploying the first and second side-branch endoprostheses includes self-expansion of the first and second side-branch endoprostheses.

## Claims

1. An endoprosthesis system comprising:
a main endoprosthesis (10) including a main flow lumen (12) and at least one side portal (14) communicating with a side channel (16), wherein the side channel (16) comprises a series of grooves (26);
a first side-branch endoprosthesis (22a) configured to be deployed within the main endoprosthesis (10) such that the first side-branch endoprosthesis (22a) extends through the side channel (16) and through a first groove (26a) of the series of grooves (26); and
a second side-branch endoprosthesis (22b) configured to be deployed within the main endoprosthesis (10) such that the second side-branch endoprosthesis (22b) extends through the side channel (16) and through a second groove (26b) of the series of grooves (26).

2. The system of claim 1, further including a third side-branch endoprosthesis (22c) configured to be deployed within the main endoprosthesis (10), the third side-branch endoprosthesis (22c) extending through the side channel (16) and through a third groove (26c) of the series of grooves (26).

3. The system of claim 1 or 2, wherein the first groove (26a) is fluidly coupled with the second groove (26b).

4. The system of any one of claims 1-3, wherein the main endoprosthesis (10) comprises a graft (18) and a stent component (20), optionally wherein the graft (18) comprises expanded polytetrafluoroethylene, optionally wherein the graft (18) comprises polyethylene, and optionally wherein the stent component (20) comprises nitinol.

5. The system of any one of claims 1-4, wherein at least one of the first side-branch endoprosthesis (22a) and second side-branch endoprosthesis (22b) is self-expanding.

6. The system of any one of claims 1-5, wherein at least one of the first side-branch endoprosthesis (22a) and second side-branch endoprosthesis (22b) is balloon-expandable.

7. The system of any one of claims 1-6, further wherein a side portal adapter (24) is positioned within the side channel (16) to divide the side channel (16) into the series of grooves (26).

8. The system of claim 7, wherein the side portal adapter (24) fluidly seals a space in the side channel (16) between the series of grooves (26).

9. The system of claim 7, wherein the side portal adapter (24) defines a central conduit (28), wherein the series of grooves (26) are connected through the central conduit (28) allowing fluid communication among the series of grooves (26).

10. The system of any one of claims 7-9, wherein the side portal adapter (24) comprises at least one of expanded polytetrafluoroethylene or polyethylene.

11. The system of any one of claims 7-10, wherein the side portal adapter (24) is attached to the main endoprosthesis (10) by at least one of adhesives, bonding, and frictional fitting.

12. The system of any one of claims 7-10, wherein the side portal adapter (24) is built into the construction of the main endoprosthesis(10).

13. The system of any one of claims 1-12, wherein the first side-branch endoprosthesis (22a) and second side-branch endoprosthesis (22b) have different diameter sizes.

14. The system of any one of claims 1-12, wherein the first side-branch endoprosthesis (22a) and second side-branch endoprosthesis (22b) have substantially the same diameter size.

15. The system of any one of claims 1-14, further including a seal to fluidly seal spaces between the side channel (16), the first side-branch endoprosthesis (22a) and the second side-branch endoprosthesis (22b), optionally wherein the seal includes a sealing cuff, an inflatable seal, an expandable seal, an adhesive, or a hydrogel.
